# EUROPÄISCHE PATENTANMELDUNG

(11) **EP 3 488 879 A1**
(43) Veröffentlichungstag der Anmeldung: **29.05.2019**
(21) Anmeldenummer: 17203246.8
(22) Anmeldetag: 23.11.2017
(51) Int. Cl.: A61M 1/00, A61M 3/02, G01F 1/56

(54) **SENSORANORDNUNG ZUM DETEKTIEREN EINER FLUIDSTRÖMUNG**

(71) Anmelder: Medela Holding AG, 6340 Baar (CH)
(72) Erfinder: BANNWART, Lukas, 6343 Rotkreuz (DE); GIEZENDANNER, Charles, 6443 Morshach (DE)
(74) Vertreter: Rutz, Andrea

(57) **Zusammenfassung**

Es wird eine Sensoranordnung zum Detektieren einer Fluidströmung bei einer Vorrichtung zum Zuführen und/oder Absaugen einer fluiden Substanz (21) zu oder von einem menschlichen oder tierischen Körper angegeben. Die Sensoranordnung weist einen Schlauch (1) mit einem Messabschnitt (12) sowie einen kapazitiven Sensor (3) zum Detektieren einer Fluidströmung im Messabschnitt (12) auf. Der Schlauch (1) hat innerhalb des Messabschnitts (12) zumindest eine Biegung (14).

## Beschreibung

### TECHNISCHES GEBIET

Die vorliegende Erfindung betrifft eine Sensoranordnung zum Detektieren einer Fluidströmung bei einer Vorrichtung zum Zuführen und/oder Absaugen einer fluiden Substanz zu oder von einem menschlichen oder tierischen Körper. Ausserdem betrifft die Erfindung eine derartige Vorrichtung zum Zuführen und/oder Absaugen einer fluiden Substanz sowie ein Anschlussteil zum Anschliessen an eine solche Vorrichtung.

### STAND DER TECHNIK

Im medizinischen Bereich gibt es vielfältige Anwendungen, bei denen Körperfluide oder Sekrete aus Körperkavitäten oder Wunden mittels einer Pumpe abgesaugt und/oder eine Substanz dem Körper zugeführt werden. Ein mögliches Anwendungsgebiet betrifft insbesondere die Unterdruck-Wundtherapie (sog. NPWT - Negative-Pressure Wound Therapy). Dabei werden Körperfluide oder Sekrete aus dem Wundbett mittels einer Pumpe abgesaugt. Während der Unterdruckbehandlung kann die Wundheilung zusätzlich durch Zuführen einer Substanz zum Wundbett (sog. Instillation) positiv beeinflusst werden. Derartige Methoden sind als Unterdruck-Wundtherapie mit kombinierter Instillation oder Irrigation bekannt. Je nach Typ und Grösse der Wunde erfolgen die Absaugung und die Zuführung dabei gleichzeitig, nacheinander und/oder abwechslungsweise intermittierend. Ein weiteres Anwendungsgebiet, bei dem fluide Substanzen dem menschlichen oder tierischen Körper zugeführt und/oder von diesem abgesaugt werden, findet sich zum Beispiel in der Augenchirurgie. Selbstverständlich gibt es auch viele medizinische Anwendungen, bei denen nur eine Absaugung von Fluiden aus dem Körper stattfindet, ohne dass eine Substanz zugeführt wird. Genauso gibt es zudem Anwendungen, bei denen nur eine Substanz dem Körper zugeführt wird, ohne dass notwendigerweise auch eine Absaugung stattfinden muss.

Falls eine fluide Substanz abgesaugt wird, kann es sich zum Beispiel um Sekrete handeln, die in einem Wundbereich vom Körper abgesondert werden, oder um Instillations- oder Spülflüssigkeit oder um beides davon.

Bei der zuzuführenden Substanz kann es sich beispielsweise um eine physiologische oder nicht physiologische Kochsalzlösung, ein Arzneimittel oder um ein Gemisch davon handeln. Die Substanz kann zum Beispiel zur Förderung der Wundheilung, zur Verhinderung von Infektionen oder zur lokalen Anästhesie dienen. Das Zuführen der Substanz kann somit zur Spülung oder therapeutischen, diagnostischen und/oder präventiven Zwecken dienen.

Aus verschiedenen Gründen kann es sowohl beim Absaugen als auch beim Zuführen von fluiden Substanzen wünschenswert sein, den Fluidfluss mittels eines oder mehreren Sensoren zu überwachen. So kann beispielsweise festgestellt werden, ob eine Absaugung bzw. Zuführung einer fluiden Substanz überhaupt stattfindet oder nicht. Bei vielen Geräten wird auch überwacht, ob es während der Absaugung bzw. Zuführung einer fluiden Substanz im entsprechenden Schlauch allenfalls eine Verstopfung gibt, um im Falle einer Verstopfung einen Alarm oder Reinigungsvorgang auszulösen.

Beispielsweise offenbart die US 5,116,203 eine Pumpe zur intravenösen Zuführung von fluiden Substanzen, bei welcher mittels Drucksensoren überwacht wird, ob eine Verstopfung vorliegt oder nicht.

Bei der in der EP 0 018 649 offenbarten Vorrichtung ist eine allfällige Verstopfung mit Hilfe von aussenseitig an einem Schlauchabschnitt anliegenden Klemmelementen detektierbar.

Die US 2015/0000423 offenbart das Anbringen von elektroaktiven Polymerstreifen an der Aussenseite eines elastischen Schlauches, um in Abhängigkeit von deren Dehnung bzw. Entspannung auf die Durchflussrate im Inneren des Schlauches schliessen zu können.

In der EP 1 834 658 ist ein Volumenstromsensor offenbart, um zum Beispiel bei einer Insulinpumpe die abgegebene Flüssigkeitsmenge zu überwachen. Hierzu sind entlang eines flexibel ausgebildeten Schlauchabschnitts einer oder mehrere Aktoren angeordnet, um eine externe Kraft auf den Schlauch auszuüben und dabei einen Rückschluss auf die Fördermenge zu erhalten.

Die US 7,462,163 offenbart ein medizinisches Infusionssystem mit einem Sensor zum Feststellen einer Verstopfung. Der Sensor ist dadurch realisiert, dass auf der Aussenseite eines gebogenen Schlauchabschnittes ein mechanischer Schalter angeordnet ist. Im Falle einer Verstopfung verdickt sich der Schlauch aufgrund des erhöhten Druckes und betätigt dadurch den Schalter.

Die oben erwähnten Sensoranordnungen des Standes der Technik sind verhältnismässig aufwändig ausgestaltet und entsprechend teuer in der Herstellung. Zu beachten ist hierbei insbesondere, dass es sich aus hygienischen Gründen beim Schlauch, durch welchen die fluide Substanz zugeführt bzw. abgesaugt wird, oftmals um einen Wegwerfartikel handelt, der entsprechend oft ausgewechselt werden muss.

Weitere Sensoranordnungen zum Detektieren von Fluidströmungen sind aus der DE 29 31 017 sowie der WO 2005/100953 bekannt. Beide Dokumente betreffen jedoch artfremde Anwendungen.

### DARSTELLUNG DER ERFINDUNG

Es ist deshalb eine Aufgabe der Erfindung, eine einfach herstellbare Sensoranordnung anzugeben, welche ein zuverlässiges Detektieren einer Fluidströmung ermöglicht.

Zur Lösung dieser Aufgabe wird eine Sensoranordnung vorgeschlagen, wie sie in Anspruch 1 angegeben ist. In Anspruch 11 wird ein Anschlussteil angegeben und in Anspruch 15 eine Vorrichtung zum Zuführen und/oder Absaugen einer fluiden Substanz zu oder von einem menschlichen oder tierischen Körper. Vorteilhafte Ausgestaltungen der Erfindung sind in den abhängigen Ansprüchen angegeben.

Die vorliegende Erfindung stellt also eine Sensoranordnung zur Verfügung zum Detektieren einer Fluidströmung bei einer Vorrichtung zum Zuführen und/oder Absaugen einer fluiden Substanz zu oder von einem menschlichen oder tierischen Körper, aufweisend
einen Schlauch mit einem Messabschnitt; sowie
einen kapazitiven Sensor zum Detektieren einer Fluidströmung im Messabschnitt, insbesondere zum Detektieren eines Unterdrucks und/oder Überdrucks einer Fluidströmung im Messabschnitt, wobei der Unterdruck tiefer liegt und der Überdruck höher liegt als ein Normaldruck, der im Betrieb der Vorrichtung während der normalen Fluidzuführung und/oder Fluidabsaugung im Messabschnitt vorliegt;
wobei der Schlauch innerhalb des Messabschnitts zumindest eine Biegung aufweist. Bei der Biegung kann es sich insbesondere um eine Krümmung, eine Ausbauchung oder um einen Knick handeln.

Da der elastisch ausgebildete Schlauch innerhalb des Messabschnitts eine Biegung aufweist, wird das im Schlauch vorhandene Fluid in der Regel aus der Biegung und somit dem Messabschnitt herausgedrückt, sobald aufgrund eines Unterdrucks keine Strömung mehr vorliegt. Der Schlauch hat dann im Messabschnitt eine erheblich kleinere Durchtrittsöffnung. Im Vergleich zur Situation, wenn eine Strömung im Messabschnitt vorliegt, das heisst Normaldruck herrscht, ist bei ausbleibender Strömung aufgrund der Biegung üblicherweise also viel weniger Fluid im Messabschnitt und somit im Messbereich des Sensors vorhanden. Im Gegensatz dazu wird die Durchtrittsöffnung des Schlauches im Bereich der Biegung bei Einsetzen einer Fluidströmung aufgeweitet, so dass Fluid in den Messabschnitt einströmen kann. Im Vergleich zur strömungsfreien Situation ist der Schlauch im Messbereich des Sensors bei Normaldruck also aufgeweitet und mit zusätzlichem Fluid gefüllt. Die somit entstehende Veränderung im Messbereich des Sensors ist messbar. Hierzu kann mittels des kapazitiven Sensors die Veränderung des Dielektrikums gemessen werden. Mit der erfindungsgemässen Vorrichtung ist es somit möglich, eine Fluidströmung im Schlauch zu detektieren. Aufgrund der Biegung ist die Detektion auch dann möglich, wenn der Druckunterschied zwischen der Situation, wenn keine Strömung vorliegt, und der Situation mit Strömung nur sehr gering ist.

Aufgrund der Biegung wird also die Durchtrittsöffnung des Schlauches, durch welche hindurch das Fluid beim Zuführen bzw. Absaugen durchströmt, in Bezug auf ihre Querschnittsfläche verkleinert. Die Biegung ist somit eine die Querschnittsfläche des Schlauches verkleinernde Biegung. Bevorzugt wird die Querschnittsfläche der Durchtrittsöffnung aufgrund der Biegung um mindestens 20%, bevorzugter um mindestens 50% und am bevorzugtesten um mindestens 70% verkleinert.

Mit dem Detektieren einer Fluidströmung ist gemeint, dass die Sensoranordnung feststellen können soll, ob eine Strömung vorliegt oder nicht, das heisst, ob ein im Schlauch vorhandenes Fluid in Bewegung ist oder stillsteht, und zwar vorteilhaft auch dann, wenn im Schlauch keine Verstopfung vorliegt, das heisst wenn im Vergleich zur Situation mit normaler Fluidströmung im Schlauch ein Unterdruck vorliegt. Die Sensoranordnung ist somit vorteilhaft dazu ausgebildet, eine Fluidströmung in Fällen zu detektieren, in denen keine Verstopfung im Schlauch vorliegt. Derartige Fälle umfassen zum Beispiel Situationen, in denen eine Fördereinrichtung zur Förderung des Fluids durch den Schlauch ausgeschaltet wird, ausfällt oder aufgrund von zum Beispiel einer Luftblase in der Zuführleitung in ihrer Funktion beeinträchtigt ist. Das Ausbleiben einer Fluidströmung kann aber auch in Fällen eintreten, bei denen der Nachschub an Fluid nicht mehr gewährleistet ist, weil sich zum Beispiel ein entsprechender Behälter zur Lagerung des Fluids leert.

Normaldruck liegt dann vor, wenn im bestimmungsgemässen Betrieb der Vorrichtung eine gewünschte Fluidströmung im Messabschnitt des Schlauchs registrierbar ist. Die Strömungsgeschwindigkeit des Fluids kann an der Vorrichtung innerhalb eines gewissen Bereiches einstellbar sein. Der Normaldruck muss somit nicht auf einen bestimmten Wert begrenzt sein, sondern kann durchaus einen gewissen Druckbereich umfassen.

Der erwähnte Normaldruck bezieht sich also ausschliesslich auf den Druck im Inneren des Schlauches während des bestimmungsgemässen Betriebes der Vorrichtung und ist somit insbesondere unabhängig vom Umgebungsdruck, welcher ausserhalb des Schlauches und ausserhalb der Sensoranordnung und der Vorrichtung herrscht. Dementsprechend bezieht sich auch der angegebene Unterdruck nicht auf den Umgebungsdruck ausserhalb der Vorrichtung, sondern auf den erwähnten Normaldruck im Inneren des Schlauches.

Dasselbe gilt auch für den Überdruck.

Des Weiteren ist es mit der Sensoranordnung bevorzugt auch möglich, das Vorliegen einer Verstopfung im Schlauch festzustellen, das heisst das Vorliegen eines Überdrucks im Schlauch zu detektieren. Wenn der Schlauch zumindest im Messabschnitt derart elastisch ausgebildet ist, dass er sich bei Vorliegen einer Verstopfung aufgrund des erhöhten Druckes noch weiter aufweitet, ist im Messabschnitt mehr Fluid vorhanden und der Schlauchumfang ist noch grösser im Vergleich zur Situation, wenn eine ungehinderte Strömung im Messabschnitt vorliegt. Die Erhöhung der Menge an Fluid im Messabschnitt bzw. der vergrösserte Schlauchumfang bei einer Verstopfung ist mittels des Sensors messbar.

Der Schlauch sollte im Messabschnitt somit bevorzugt derart elastisch ausgebildet sein, dass aufgrund der Biegung der Schlauchumfang abnimmt und/oder die Menge an Fluid im Messabschnitt dadurch deutlich geringer ist, wenn wegen eines Unterdrucks keine Fluidströmung vorliegt als wenn eine Fluidströmung bei Normaldruck vorhanden ist. Zudem sollte der Schlauch im Messabschnitt vorteilhaft derart elastisch ausgebildet sein, dass sich seine Durchtrittsöffnung aufgrund des erhöhten Druckes aufweitet und somit die Menge an Fluid im Messabschnitt deutlich erhöht ist, wenn eine Verstopfung vorliegt. Der Schlauch weist vorteilhaft einen Elastizitätsmodul von 1 bis 1000 N/mm², noch vorteilhafter von 10 bis 100 N/mm² auf. Beim Material des Schlauches handelt es sich vorzugsweise um Silikon oder ein thermoplastisches Elastomer.

Aufgrund der Biegung und der Elastizität des Schlauches im Messabschnitt sowie aufgrund des kapazitiven Sensors ist die Sensoranordnung also bevorzugt dazu ausgebildet, sämtliche der folgenden vier Situationen voneinander zu unterscheiden und somit zu detektieren:
- Im Messabschnitt ist kein oder nur wenig Fluid vorhanden;
- Im Messabschnitt ist Fluid vorhanden, das jedoch stillsteht;
- Im Messabschnitt liegt eine normale, d.h. bestimmungsgemässe Fluidströmung vor;
- Im fluidführenden System, insbesondere im Schlauch, liegt eine Verstopfung vor.

Beim Fluid handelt es sich in der Regel um eine fliessfähige Substanz wie zum Beispiel eine Flüssigkeit, ein Pulver oder ein Gemisch davon. Es kann sich zum Beispiel um Sekrete handeln, die in einem Wundbereich vom Körper abgesondert werden, oder um Instillations- oder Spülflüssigkeit oder um beides davon.

Die Vorrichtung zum Zuführen und/oder Absaugen einer fluiden Substanz zu oder von einem menschlichen oder tierischen Körper kann insbesondere eine reine Drainagevorrichtung, eine reine Instillationsvorrichtung oder eine kombinierte Vorrichtung sein, welche für die Unterdruck-Wundtherapie mit kombinierter Instillation oder Irrigation ausgebildet ist. Vorzugsweise dient die Vorrichtung zur Wundtherapie. Sie kann aber auch für die Augenchirurgie oder eine beliebige andere, bevorzugt medizinische Anwendung ausgebildet sein.

Der kapazitive Sensor weist üblicherweise zumindest eine, vorteilhaft zumindest zwei Elektroden auf, welche vorteilhaft jeweils plattenförmig ausgebildet sind. Die vorteilhaft zumindest zwei Elektroden sind bevorzugt mit einer Kontrolleinheit verbunden, welche eine elektrische Spannung zwischen den zumindest zwei Elektroden erzeugen kann, um die Kapazität der Elektrodenanordnung zu messen. Das dabei zwischen den Elektroden erzeugte elektrische Feld umfasst zumindest den Messabschnitt des Schlauches derart, dass eine Dielektrikumsveränderung aufgrund von unterschiedlichen Mengen an Fluid im Messabschnitt messbar ist. Im Falle, dass der kapazitive Sensor nur eine einzige Elektrode aufweist, kann die den Messabschnitt umgebende Luft als Gegenelektrode verwendet werden.

Bevorzugt weist der kapazitive Sensor zumindest zwei ausserhalb des Schlauches angeordnete Elektroden auf. Die zumindest zwei ausserhalb des Schlauches angeordneten Elektroden bilden vorzugsweise ein Paar in dem Sinne, dass zur Kapazitätsmessung eine Spannung zwischen den beiden Elektroden anlegbar ist. Das heisst, die bei einer Messung entstehenden Feldlinien erstrecken sich vorzugsweise von der ersten zur zweiten Elektrode dieses Paars.

Vorteilhaft ist sogar der gesamte kapazitive Sensor ausserhalb des Schlauches angeordnet. Dies ergibt eine besonders einfach herstellbare Ausführungsform, da der Schlauch getrennt vom kapazitiven Sensor produzierbar ist. Es kann dadurch ein herkömmlicher Schlauch verwendet werden, welcher nach Gebrauch auch einfach austauschbar ist.

Der Schlauch kann im Messabschnitt auch mehr als eine Biegung aufweisen. Dadurch kann insbesondere der vom Schlauch verursachte Druck zum Herausdrücken des Fluids aus dem Messabschnitt erhöht werden.

Der kapazitive Sensor kann mehr als zwei Elektroden, insbesondere mehr als zwei Paare von Elektroden aufweisen. Mittels einer hohen Anzahl von Elektroden kann die Messgenauigkeit und/oder die Ausfallsicherheit, im Hinblick darauf, wenn zum Beispiel einzelne oder mehrere Elektroden defekt sind, erhöht werden. Wenn der Messabschnitt mehrere Biegungen aufweist, kann insbesondere jeweils ein Paar von Elektroden einer Biegung zugeordnet sein.

In der zumindest einen Biegung ist der Schlauch bevorzugt um mindestens 30°, vorteilhaft um mindestens 50° und am vorteilhaftesten um mindestens 80° umgebogen. Bei der Biegung kann es sich insbesondere um einen Knick im Schlauch handeln. Bei einem Knick ist die Durchtrittsöffnung des Schlauches für das Fluid an der entsprechenden Stelle, wenn keine Fluidströmung vorliegt, auf ein Minimum verringert oder sogar im Wesentlichen ganz verschlossen.

Bevorzugt weist die Sensoranordnung zusätzlich ein Umlenkelement auf zur Herstellung der Biegung. Das Umlenkelement dient insbesondere dazu, dass der Schlauch im Messabschnitt eine Biegung aufweist und in dieser Position gehalten wird. Das Umlenkelement kann eine kreisbogenförmige Fläche bilden, welche sich vorteilhaft über einen Winkelbereich von mindestens 45°, vorteilhafter von mindestens 60° und am vorteilhaftesten von mindestens 90° erstreckt, und an welcher der Schlauch im Bereich der Biegung anliegt. Die kreisbogenförmige Fläche dient dann dazu, die Form des Schlauches im Bereich der Biegung festzulegen. Vorteilhaft ist pro Biegung jeweils ein Umlenkelement vorhanden.

Gemäss einer Weiterbildung der Erfindung ist das Umlenkelement bevorzugt relativ zu derjenigen Stelle bewegbar, wo der Schlauch die zumindest eine Biegung aufweist. Zur Lagerung ist das Umlenkelement somit bevorzugt derart vom Schlauch weg bewegbar, dass die Biegung aufgehoben oder zumindest verringert wird. Das Schlauchmaterial wird dadurch entlastet, so dass sich die Lebensdauer des Schlauches erhöht.

Die Sensoranordnung kann einen Anschlussstutzen zum Anschliessen des Schlauches aufweisen, wobei der Anschlussstutzen derart in der Nähe des Umlenkelements angeordnet und auf dieses hin ausgerichtet ist, dass das Umlenkelement beim Anschliessen des Schlauches am Anschlussstutzen eine Biegung, insbesondere Knick, des Schlauches bewirkt. Beim Umlenkelement kann es sich um einen beliebigen Anschlag für den Schlauch handeln, der zum Beispiel auch durch eine Gehäusewand oder ein Abdeckteil gebildet sein kann. Bevorzugt erstreckt sich der Anschlussstutzen entlang seiner Längsmittelachse geneigt zu einer Wandung, innerhalb welcher er angeordnet ist.

Gemäss einer Weiterbildung der Erfindung kann die Biegung des Schlauches im Messabschnitt durch die Anpressrolle einer Peristaltikpumpe bewirkt sein. Der Pumpenkopf der Peristaltikpumpe mit den Anpressrollen kann einen Teil der Sensoranordnung bilden. Bei Verwendung einer Peristaltikpumpe kann dadurch auf eine besonders einfache Art und Weise der Fluidströmung detektiert werden. Im Betrieb der Peristaltikpumpe wird dann vom Sensor ein periodisches, intermittierendes Signal gemessen. Da sich dieses Signal bei Ausbleiben einer Fluidströmung bzw. bei einer Verstopfung im Schlauch verändert, kann eine entsprechende Detektion der Fluidströmung erfolgen.

Die vorliegende Erfindung bezieht sich ausserdem auf ein Anschlussteil zum Anschliessen an eine Vorrichtung zum Zuführen und/oder Absaugen einer fluiden Substanz zu oder von einem menschlichen oder tierischen Körper, aufweisend einen Schlauch sowie eine Schlauchführung zum Führen des Schlauches derart, dass der Schlauch zumindest eine Biegung aufweist und dadurch einen Messabschnitt zum Detektieren einer Fluidströmung, insbesondere eines Unterdrucks in einer Fluidströmung mittels eines kapazitiven Sensors bildet. Die Schlauchführung ist bevorzugt dazu ausgebildet, zusammen mit dem Sensor eine Sensoranordnung entsprechend der obigen Ausführungen zu bilden.

Die Vorrichtung zum Zuführen und/oder Absaugen einer fluiden Substanz kann insbesondere eine reine Drainagevorrichtung, eine reine Instillationsvorrichtung oder eine kombinierte Vorrichtung sein, welche für die Unterdruck-Wundtherapie mit kombinierter Instillation oder Irrigation ausgebildet ist. Vorzugsweise weist die Vorrichtung zum Zuführen und/oder Absaugen einer fluiden Substanz ein Gehäuse mit einem darin untergebrachten Antrieb, wie insbesondere einen Elektromotor, auf. Der Antrieb dient bevorzugt zum Antreiben einer Pumpe, deren Pumpenkopf im oder am Gehäuse oder in oder am Anschlussteil angeordnet sein kann.

Beim Anschlussteil kann es sich insbesondere um einen Fluidsammelbehälter zur Aufnahme der abgesaugten fluiden Substanz handeln. Das Anschlussteil kann aber auch zum Anschliessen an einen Fluidsammelbehälter, an ein Pumpengehäuse und/oder an eine Sensoreinheit ausgebildet sein. Das Anschliessen kann zum Beispiel mittels Einrasten, Einschnappen, Einspannen, Verkleben oder Verschrauben erfolgen.

Der Schlauch ist bevorzugt dazu ausgebildet, einen Teil einer Drainageleitung, einer Hilfsleitung oder einer Instillationsleitung zu bilden.

Der Schlauch kann in das Innere eines Fluidsammelbehälters münden und dadurch als Teil einer Drainage- oder Absaugleitung vorgesehen sein, durch welche hindurch die abgesaugte fluide Substanz in den Fluidsammelbehälter hinein gefördert wird. Mittels der Sensoranordnung ist es dann möglich, die Menge der bereits abgesaugten und somit im Fluidsammelbehälter gesammelten fluiden Substanz zu bestimmen. Dies ist insbesondere dann vorteilhaft, wenn im Fluidsammelbehälter ein Gelier- oder Verdickungsmittel vorhanden ist, um abgesaugte Sekrete zu binden.

Das Anschlussteil kann zusätzlich eine Peristaltikpumpeneinheit, insbesondere einen Pumpenkopf einer Peristaltikpumpe, aufweisen und der Schlauch kann ein Teil dieser Peristaltikpumpeneinheit darstellen, so dass die fluide Substanz mittels der Peristaltikpumpeneinheit durch den Schlauch hindurch zum menschlichen oder tierischen Körper hin gefördert oder von diesem abgesaugt werden kann.

Des Weiteren bezieht sich die Erfindung auf eine Vorrichtung zum Zuführen und/oder Absaugen einer fluiden Substanz zu oder von einem menschlichen oder tierischen Körper, aufweisend
ein Pumpengehäuse zur Aufnahme eines Pumpenantriebs, insbesondere ein Saugpumpengehäuse zur Aufnahme eines Saugpumpenantriebs oder einer Saugpumpe; sowie
ein Anschlussteil, insbesondere einen Fluidsammelbehälter, entsprechend den obigen Ausführungen, zum Anschliessen an das Pumpengehäuse.

Die Vorrichtung zum Zuführen und/oder Absaugen einer fluiden Substanz weist zudem vorteilhaft einen kapazitiven Sensor zum Detektieren einer Fluidströmung im Messabschnitt des erwähnten Schlauches des Anschlussteils auf. Die Elektroden des kapazitiven Sensors sind vorteilhaft auf einer Innenseite des Pumpengehäuses angeordnet. Der kapazitive Sensor ist dadurch optimal vor externen Einflüssen geschützt.

Bei einer insbesondere bevorzugten Ausführungsform weist das Pumpengehäuse ein Umlenkelement auf, welches beim Anschliessen des Anschlussteils an das Pumpengehäuse die zumindest eine Biegung des Schlauches bewirkt. Auf diese Weise ist sichergestellt, dass der Schlauch erst dann im Messabschnitt umgebogen und somit belastet wird, wenn das Anschlussteil am Pumpengehäuse angeschlossen ist.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1a: eine nicht erfindungsgemässe Sensoranordnung mit einem kapazitiven Sensor und einem mit Luft gefüllten Schlauchabschnitt;
- Fig. 1b: die Sensoranordnung der Fig. 1a, wobei der Schlauchabschnitt mit einem Fluid gefüllt ist;
- Fig. 1c: die Sensoranordnung der Fig. 1a, wobei der Schlauchabschnitt eine Verstopfung aufweist;
- Fig. 2a: eine Draufsicht auf eine Schlauchführungsvorrichtung einer Sensoranordnung gemäss einer ersten erfindungsgemässen Ausführungsform, bei nicht vorhandener Fluidströmung;
- Fig. 2b: eine Draufsicht auf die Schlauchführungsvorrichtung der Fig. 2a, bei vorhandener Fluidströmung;
- Fig. 2c: eine Draufsicht auf die Schlauchführungsvorrichtung der Fig. 2a, bei Verstopfung;
- Fig. 3a: eine perspektivische Ansicht der in der Fig. 2a gezeigten Schlauchführungsvorrichtung;
- Fig. 3b: eine perspektivische Ansicht der in der Fig. 2b gezeigten Schlauchführungsvorrichtung;
- Fig. 3c: eine perspektivische Ansicht der in der Fig. 2c gezeigten Schlauchführungsvorrichtung;
- Fig. 4a: eine perspektivische Ansicht einer Sensoranordnung gemäss einer zweiten erfindungsgemässen Ausführungsform, bei nicht vorhandener Fluidströmung;
- Fig. 4b: eine perspektivische Ansicht der Sensoranordnung der Fig. 4a, bei vorhandener Fluidströmung;
- Fig. 4c: eine perspektivische Ansicht der Sensoranordnung der Fig. 4a, bei Verstopfung;
- Fig. 5a: eine perspektivische Ansicht der Schlauchführungsvorrichtung der Sensoranordnung der Fig. 4a, mit geöffnetem Einspannhebel;
- Fig. 5b: eine perspektivische Ansicht der Schlauchführungsvorrichtung der Sensoranordnung der Fig. 4a, mit geschlossenem Einspannhebel;
- Fig. 6a: eine perspektivische Ansicht eines schematisch dargestellten Fluidsammelbehälters inklusive Peristaltikpumpenkopf und Schlauchführungsvorrichtung einer Sensoranordnung gemäss einer dritten erfindungsgemässen Ausführungsform;
- Fig. 6b: eine perspektivische Ansicht des Fluidsammelbehälters der Fig. 6a aus einer anderen Richtung;
- Fig. 6c: eine perspektivische Ansicht eines schematisch dargestellten Saugpumpengehäuses mit einem kapazitiven Sensor, an welches der Fluidsammelbehälter der Fig. 6a anschliessbar ist;
- Fig. 7a: eine perspektivische Ansicht eines schematisch dargestellten Fluidsammelbehälters mit einer Schlauchführung einer Sensoranordnung gemäss einer vierten erfindungsgemässen Ausführungsform;
- Fig. 7b: eine perspektivische Ansicht eines schematisch und nur teilweise dargestellten Saugpumpengehäuses mit einem kapazitiven Sensor, an welches der Fluidsammelbehälter der Fig. 7a anschliessbar ist;
- Fig. 8a: eine perspektivische Ansicht einer Schlauchführungsvorrichtung für eine Sensoranordnung gemäss einer weiteren erfindungsgemässen Ausführungsform; sowie
- Fig. 8b: die Schlauchführungsvorrichtung der Fig. 8a, ohne Schlauch.

### BESCHREIBUNG BEVORZUGTER AUSFÜHRUNGSFORMEN

Im Folgenden sind bei unterschiedlichen Ausführungsformen gleiche oder ähnliche Merkmale, welche eine gleiche oder ähnliche Funktion erfüllen, jeweils mit denselben Bezugszeichen versehen.

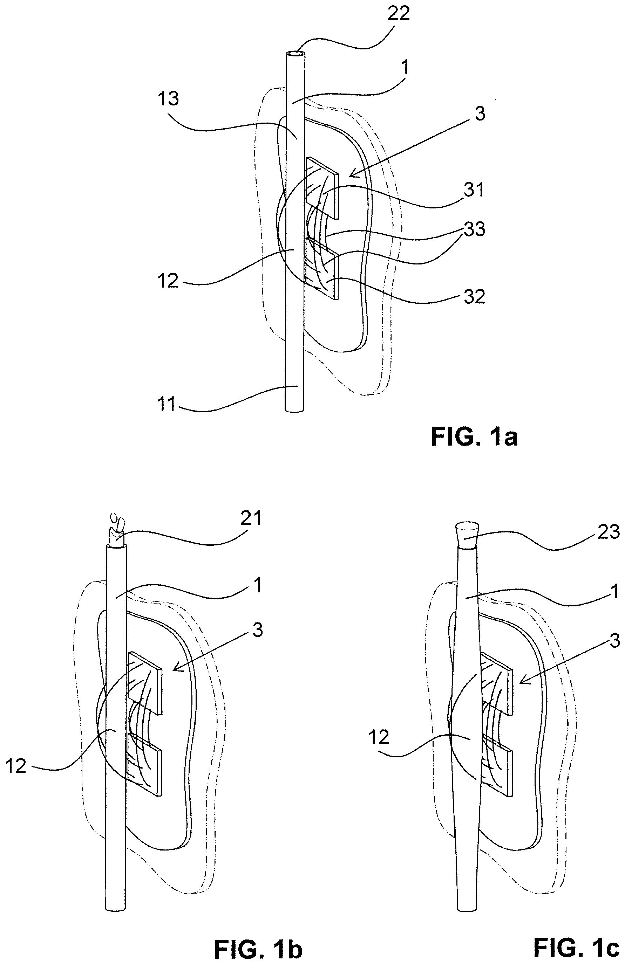

In den Figuren 1a bis 1c ist eine nicht erfindungsgemässe Sensoranordnung zum Detektieren einer Fluidströmung gezeigt. Die Sensoranordnung ist für den Einsatz bei einer Vorrichtung zum Zuführen und/oder Absaugen einer fluiden Substanz zu oder von einem menschlichen oder tierischen Körper ausgebildet.

Wie in der Figur 1a gut erkennbar ist, weist die Sensoranordnung einen kapazitiven Sensor 3 auf, welcher im Bereich eines Messabschnittes 12 eines Schlauches 1 angeordnet ist. Der Messabschnitt 12 ist zwischen einem Eingangsabschnitt 11 und einem Ausgangsabschnitt 13 des Schlauches 1 angeordnet. Der Schlauch 1 kann zum Bespiel ein Teil einer Drainageleitung zum Absaugen von Körpersekreten aus einer Wunde bilden oder ein Teil einer Zuführleitung, durch welche hindurch einem menschlichen oder tierischen Körper Medikamente oder eine Spülflüssigkeit zugeführt werden.

Der kapazitive Sensor 3 weist eine erste Elektrode 31 und eine zweite Elektrode 32 auf, zwischen denen bei entsprechender elektrischer Ansteuerung ein elektrisches Feld ausgebildet werden kann. Je nach Höhe der zwischen den Elektroden 31 und 32 gemessenen Kapazität kann auf das sich dazwischen befindliche Dielektrikum geschlossen werden. Das Dielektrikum verändert sich dabei in Abhängigkeit der im Messabschnitt 12 vorhandenen Menge an Fluid 21 (siehe Figur 1b). In den Figuren 1a bis 1c ist das zwischen den Elektroden 31, 32 ausgebildete elektrische Feld mittels Feldlinien 33 dargestellt.

Wenn aufgrund des Absaug- oder Zuführvorgangs ein Fluid 21 in den zuvor ausschliesslich mit Luft 22 gefüllten Schlauch 1 gelangt (Figuren 1a bzw. 1b), verändert sich das zwischen den Elektroden 31, 33 gemessene Dielektrikum, so dass mittels des kapazitiven Sensors 3 die Abwesenheit bzw. das Vorhandensein des Fluids 21 in der Leitung feststellbar ist.

In der Figur 1c ist die Situation bei einer Verstopfung des Schlauches 23 gezeigt, was mit einem Pfropfen 23 versinnbildlicht wird. Aufgrund des durch die Verstopfung erhöhten Druckes weitet sich der flexibel ausgebildete Schlauch 1 im Bereich des Messabschnittes 12 auf, wodurch das vom kapazitiven Sensor 3 gemessene Dielektrikum im Vergleich zu der in der Figur 1b dargestellten Situation nochmals verändert wird. Eine Verstopfung des Schlauches 1 ist mit der in den Figuren 1a bis 1c gezeigten Sensoranordnung somit detektierbar.

Mit der in den Figuren 1a bis 1c dargestellten Sensoranordnung lässt sich jedoch nicht feststellen, ob in der Situation der Figur 1b ein Fluidstrom vorliegt, oder ob das Fluid 21 im Messabschnitt 12 aufgrund eines Unterdrucks stillsteht, das heisst ob in Bezug auf das Fluid 21 eine Strömung vorliegt oder nicht.

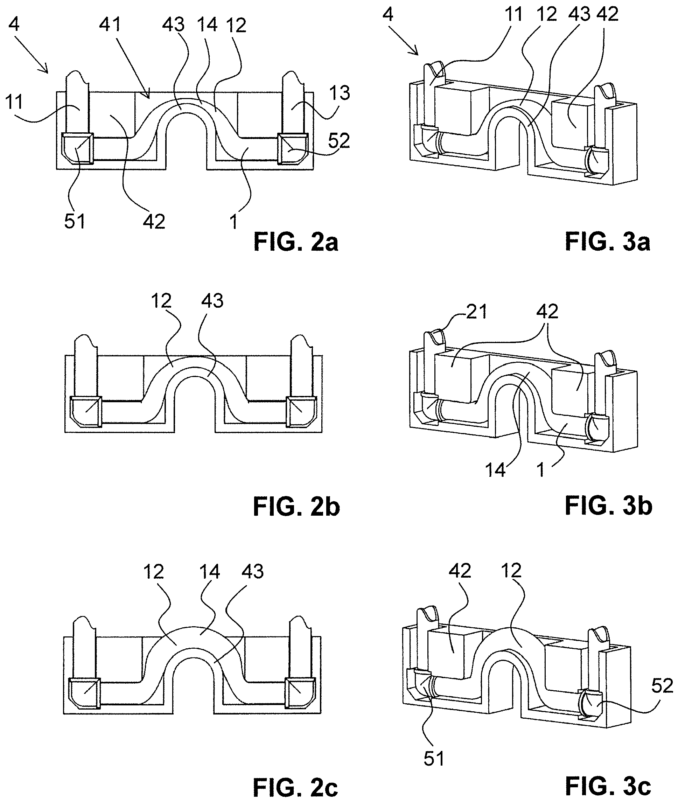

In den Figuren 2a bis 3c ist eine Schlauchführungsvorrichtung 4 gezeigt, welche einen ersten Teil einer Sensoranordnung gemäss einer ersten erfindungsgemässen Ausführungsform bildet. Ein zweiter Teil dieser Sensoranordnung wird durch einen in den Figuren 2a bis 3c nicht dargestellten Sensor gebildet, bei dem es sich ähnlich wie bei der in den Figuren 1a bis 1c gezeigten Ausführungsform um einen kapazitiven Sensor 3 handeln kann. Bei Verwendung eines kapazitiven Sensors ist die Detektion der Fluidströmung besonders einfach.

Wie in der Figur 2a ersichtlich ist, weist die Schlauchführungsvorrichtung 4 einen Schlauch 1 mit einem Eingangsabschnitt 11, einem Messabschnitt 12 und einem Ausgangsabschnitt 13 auf. Zwischen dem Eingangsabschnitt 11 und dem Messabschnitt 12 einerseits sowie zwischen dem Messabschnitt 12 und dem Ausgangsabschnitt 13 andererseits, ist jeweils ein erstes bzw. zweites Rohrwinkelstück 51 bzw. 52 angeordnet. Die Rohrwinkelstücke 51 und 52 dienen dazu, den Schlauch 1 jeweils rechtwinklig umzulenken, so dass sich der Eingangsabschnitt 11 und der Ausgangsabschnitt 13 parallel und nebeneinander erstrecken können. Der Schlauch 1 bildet dadurch im Bereich der Schlauchführungsvorrichtung 4 eine U-Form. Die Rohrwinkelstücke 51, 52 unterbrechen den Schlauch 1 zwar an den entsprechenden Stellen jeweils, bilden aber jeweils eine dichte Fluidverbindung zwischen dem Eingangsabschnitt 11 und dem Messabschnitt 12 sowie dem Messabschnitt 12 und dem Ausgangsabschnitt 13. Aufgrund der Rohrwinkelstücke 51 und 52 wird der Schlauch 1 an den beiden Umlenkstellen entlastet. Grundsätzlich können die Rohrwinkelstücke 51 und 52 aber auch entfallen, und der Schlauch 1 könnte an den entsprechenden Stellen einfach rechtwinklig umgebogen sein.

Die Schlauchführungsvorrichtung 4 weist eine Schlauchführung 41 zur genau festgelegten Führung des Schlauches 1 insbesondere im Bereich seines Messabschnittes 12 auf. Hierzu sind Führungselemente 42 sowie ein Umlenkelement 43 vorgesehen, welche eine Biegung 14 des Schlauches 1 im Messabschnitt 12 verursachen. Der Schlauch 1 wird dabei im Messabschnitt 12 um annähernd 90° umgebogen. Er erstreckt sich über einen Winkelbereich von etwas mehr als 90° entlang eines Kreisbogens, der durch das Umlenkelement 43 gebildet wird.

Genauso wie bei der in den Figuren 1a bis 1c gezeigten Ausführungsform ist der Messabschnitt 12 und somit die Biegung 14 des Schlauches bei der bevorzugten Verwendung eines kapazitiven Sensors innerhalb der Feldlinien des in den Figuren 2a bis 3c nicht dargestellten kapazitiven Sensors angeordnet.

Bei einer unterdruckbedingten nicht vorhandenen Fluidströmung im Schlauch 1 wird allfälliges im Messabschnitt 12 verbleibendes Fluid 21 aufgrund der Elastizität des Schlauches 1 aus der Biegung 14 herausgedrückt und in den Eingangsabschnitt 11 sowie den Ausgangsabschnitt 13 verschoben. Im Messabschnitt 12 und insbesondere in der Biegung 14 ist dann somit gar kein oder nur sehr wenig Fluid vorhanden. Diese Situation ist in den Figuren 2a und 3a gezeigt.

Wenn aufgrund des Betriebs von zum Beispiel einer Pumpe eine Fluidströmung im Schlauch 1 vorhanden ist, wird die Durchtrittsöffnung des Schlauches 1 im Bereich der Biegung 14 aufgrund der Erhöhung des Druckes auf Normaldruck und des durchströmenden Fluids 21 aufgeweitet, wie in den Figuren 2b und 3b dargestellt ist. Im Vergleich zur Situation der Figuren 2a und 3a verändert sich dadurch die vom kapazitiven Sensor gemessene Kapazität erheblich. Mit der in den Figuren 2a bis 3c gezeigten Sensoranordnung ist es somit im Gegensatz zur der in den Figuren 1a bis 1c gezeigten Ausführungsform möglich zu detektieren, ob im Schlauch 1 eine Fluidströmung vorliegt, oder ob das Fluid 21 stillsteht. Dies wird durch das Vorsehen der Biegung 14 innerhalb des Messabschnitts 12 erreicht.

Im Falle einer Verstopfung weitet sich die Durchtrittsöffnung des Schlauches 1 im Bereich der Biegung 14 aufgrund des dann vorliegenden Überdruckes noch weiter auf (Figuren 2c und 3c). Es befindet sich dadurch eine noch grössere Menge an Fluid 21 im Messabschnitt 12, was durch den kapazitiven Sensor im Vergleich zur Situation der Figuren 2b und 3b feststellbar ist.

Somit lässt sich mit der in den Figuren 2a bis 3c dargestellten Sensoranordnung nicht nur feststellen, ob eine Verstopfung vorliegt oder nicht, sondern auch eine zuverlässige Detektion einer Fluidströmung ist dank der Biegung 14 möglich.

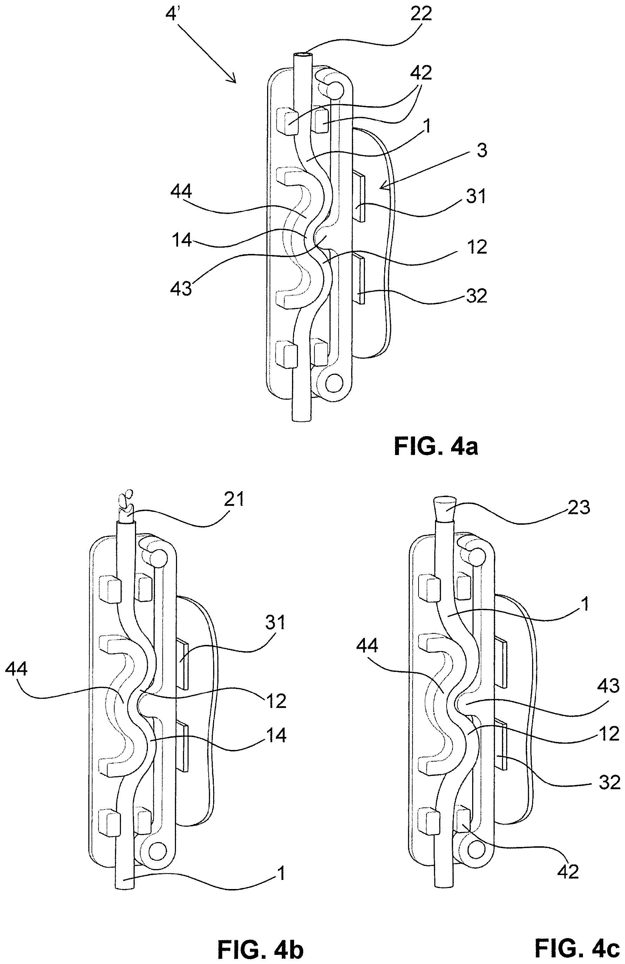

Eine weitere Ausführungsform einer erfindungsgemässen Sensoranordnung ist in den Figuren 4a bis 5b gezeigt. Wie die Ausführungsform der Figuren 2a bis 3c, weist auch die Sensoranordnung der Figuren 4a bis 5b eine Schlauchführungsvorrichtung 4' sowie bevorzugt einen kapazitiven Sensor 3 auf. Zudem wird auch hier bei einem Schlauch 1 innerhalb eines Messabschnitts 12 mit Hilfe eines Umlenkelements 43 eine Biegung 14 bewirkt, welche in dem von den Elektroden 31, 32 des kapazitiven Sensors 3 erfassten Messbereich zu liegen kommt.

Auf der dem Umlenkelement 43 gegenüberliegenden Seite des Schlauches 1 ist bei der Ausführungsform der Figuren 4a bis 5b eine Bogenführung 44 vorgesehen. Die Bogenführung 44 definiert die Erstreckungsrichtung des Schlauches 1 auf der vom Umlenkelement 43 gegenüberliegenden Seite. Vor und nach der Bogenführung 44 wird der Schlauch 1 mittels seitlicher Führungselemente 44 geführt.

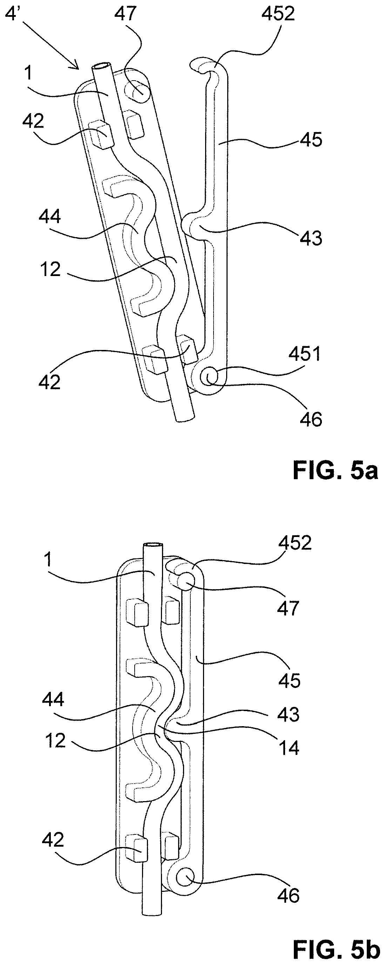

Um den Schlauch 1 während der Lagerung zu entlasten, weist die Schlauchführungsvorrichtung 4' der Figuren 4a bis 5b einen Einspannhebel 45 auf, an welchem das Umlenkelement 43 angebracht ist (siehe insbesondere die Figuren 5a und 5b). Der Einspannhebel 45 ist gelenkig an demjenigen Teil der Schlauchführungsvorrichtung 4' angebracht, welches die Bogenführung 44 und die Führungselemente 42 aufweist. Hierzu ist der Einspannhebel 45 via eine Zapfenaufnahme 451 schwenkbar an einem Gelenkzapfen 46 angebracht. An seinem der Zapfenaufnahme 451 gegenüberliegenden Endbereich weist der Einspannhebel 45 einen Einrasthaken 425 auf, welcher im geschlossenen Zustand des Einspannhebels 45 (Figur 5b) an einem Einrastzapfen 47 einrastbar ist. Der Einrastzapfen 47 ist ortsfest relativ zur Bogenführung 44 und den Führungselementen 42 an der Schlauchführungsvorrichtung 4' angeordnet.

Für die Lagerung der Schlauchführungsvorrichtung 4' wird der Einspannhebel 45 in seine geöffnete Position entsprechend der Figur 5a gebracht. Der Einspannhebel 45 ist dann nicht mehr mit dem Einrastzapfen 47 verrastet, sondern relativ zur Bogenführung 44 weg verschwenkt. Dabei wird insbesondere die vom Umlenkelement 43 auf den Schlauch 1 ausgeübte Kraft verringert oder sogar ganz aufgehoben. Der Schlauch 1 weist demzufolge im Bereich des Messabschnittes 12 keine Biegung 14 mehr auf, sondern verläuft weitgehend geradlinig. Das Schlauchmaterial wird dadurch entlastet und die Lebensdauer des Schlauches 1 erhöht.

Bei Benutzung der Schlauchführungsvorrichtung 4' wird der Einspannhebel 45 jedoch in seine geschlossene Position entsprechend der Figur 5b gebracht. Der Einrasthaken 452 ist dann am Einrastzapfen 47 verrastet, und das Umlenkelement 43 übt eine Kraft auf den Schlauch 1 im Messabschnitt 12 aus, wodurch die Biegung 14 entsteht.

Die Schlauchführungsvorrichtung 4 der Figuren 2a bis 3c sowie die Schlauchführungsvorrichtung 4' der Figuren 4a bis 5b können beispielsweise ein Teil einer Saugpumpeneinheit bilden, um in Kombination mit einem kapazitiven Sensor 3 den Absaugvorgang durch eine Drainageleitung hindurch zu überwachen. Der Schlauch 1 würde dann einen Teil der Drainageleitung bilden. Alternativ könnten die Schlauchführungsvorrichtungen 4, 4' zusammen mit einem kapazitiven Sensor 3 auch zur Überwachung eines Instillationsvorganges benutzt werden, wobei der Schlauch 1 dann einen Teil der Instillationsleitung bildet. Die Instillation kann dabei herkömmlich, das heisst unter Ausnutzung des hydrostatischen Druckes mittels eines erhöht angeordneten Behälters, oder mit Hilfe einer Pumpe, insbesondere einer Peristaltikpumpe, durchgeführt werden. Bei Verwendung einer Pumpe können die Schlauchführungsvorrichtung 4, 4' sowie der kapazitive Sensor 3 insbesondere am Pumpengehäuse angebracht sein.

Die in den Figuren 2a bis 5b gezeigten Schlauchführungsvorrichtungen 4, 4' können aber auch zum Nachrüsten für bestehende Absaug- und/oder Zuführvorrichtungen vorgesehen sein, bei denen beispielweise bereits eine Sensoranordnung mit insbesondere einem kapazitiven Sensor entsprechend den Figuren 1a bis 1c vorhanden ist. Durch das Nachrüsten kann die Funktionalität der Sensoranordnung erweitert bzw. verändert werden. Die Schlauchführungsvorrichtungen 4, 4' können somit Anschlussteile zum Anschliessen an bestehende Vorrichtungen bilden, wobei das Anschliessen zum Beispiel mittels Einrasten, Verkleben oder Verschrauben möglich ist.

Unabhängig davon, ob die Anwendung die Überwachung des Zuführ- oder des Absaugvorgangs betrifft, können die Schlauchführungsvorrichtungen 4 und 4' anstatt am Pumpengehäuse auch an einem Fluidsammelbehälter, welcher zum Sammeln der abgesaugten Fluide vorgesehen ist, oder an einem Reservoir angebracht sein, in welchem z.B. die Instillationsflüssigkeit gelagert ist.

Besonders vorteilhaft ist es, wenn die Schlauchführungsvorrichtung 4 bzw. 4' an einem Fluidsammelbehälter oder einem Reservoir und der kapazitive Sensor 3 an einem Saugpumpengehäuse angebracht sind, an welches der Fluidsammelbehälter bzw. das Reservoir anschliessbar ist. Dies deshalb, weil der Fluidsammelbehälter bzw. das Reservoir sowie der Schlauch 1 aus hygienischen Gründen oftmals Wegwerfartikel darstellen, welche nach einmaligem Gebrauch bereits entsorgt bzw. ausgetauscht werden müssen. Der elektrisch mit einer Kontrolleinheit verbundene kapazitive Sensor 3 ist dann als Teil des Saugpumpengehäuses vom Austausch nicht betroffen.

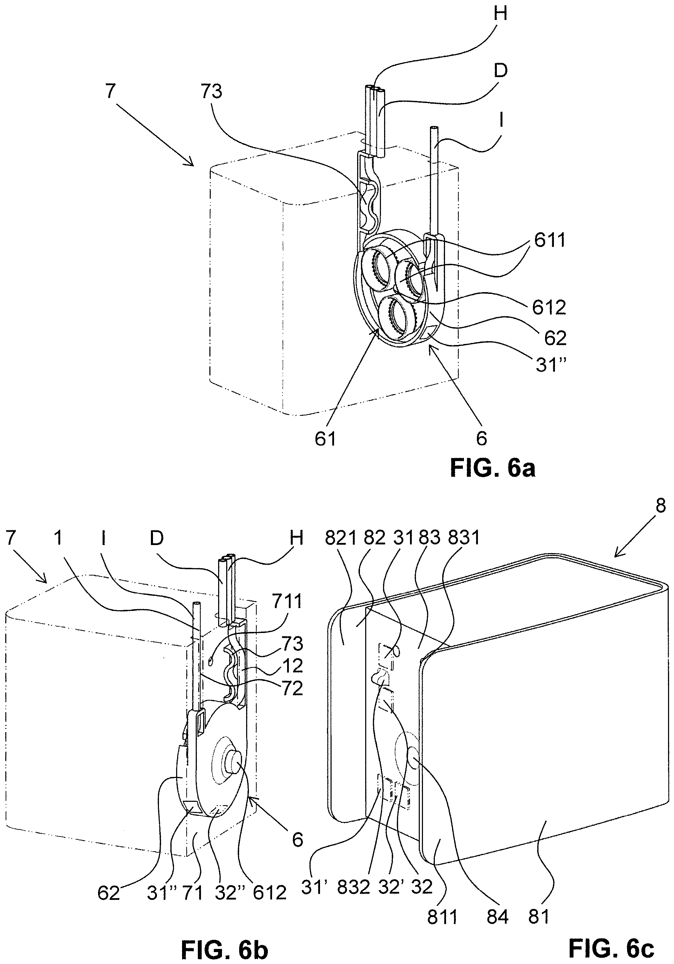

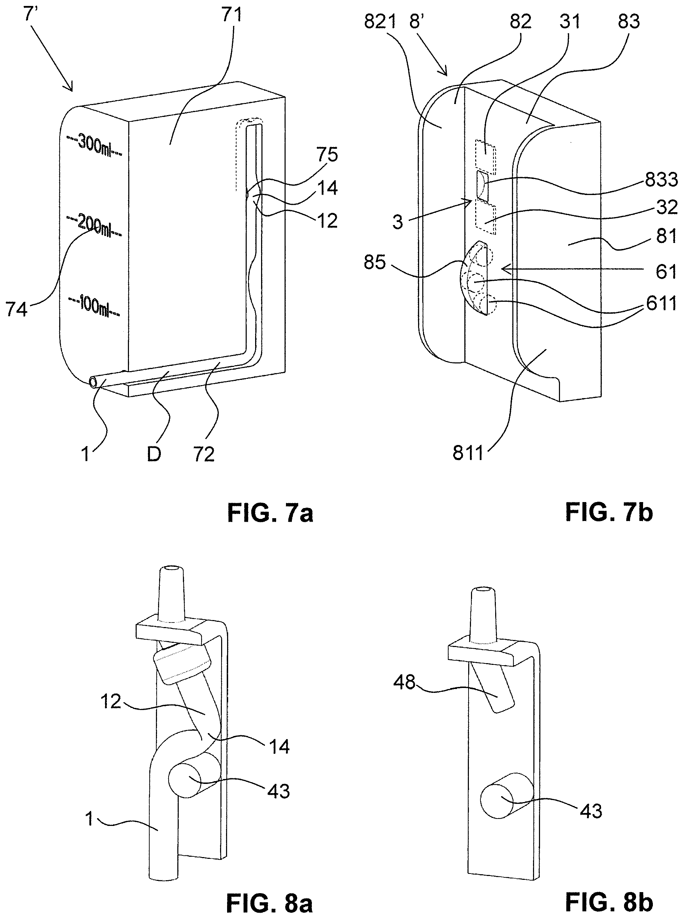

In den Figuren 6a bis 6c sowie 7a und 7b sind Ausführungsformen gezeigt, bei denen ein Schlauch 1 jeweils in einer an einem Fluidsammelbehälter 7 bzw. 7' vorhandenen Schlauchführung derart geführt ist, dass er in einem Messabschnitt 12 eine Biegung 14 aufweist. Der Fluidsammelbehälter 7 bzw. 7' ist jeweils derart an ein Saugpumpengehäuse 8 bzw. 8' anschliessbar, dass der Messabschnitt 12 des Schlauches 1 im Bereich eines am Saugpumpengehäuse 8 bzw. 8' angeordneten kapazitiven Sensors 3 mit Elektroden 31, 32 zu liegen kommt.

Bei der in den Figuren 6a bis 6c gezeigten Ausführungsform handelt es sich um eine Vorrichtung für die mit Instillation kombinierte Unterdruck-Wundtherapie. Mit Hilfe einer im Saugpumpengehäuse 8 untergebrachten Pumpe können Sekrete durch eine Drainageleitung D hindurch von der Wunde eines Patienten in den Fluidsammelbehälter 7 hinein abgesaugt werden. Die Drainageleitung D mündet hierzu in das Innere des Fluidsammelbehälters 7.

Der Fluidsammelbehälter 7 ist zwischen zwei vorstehenden Wandkanten 811 und 821 einer Vorderwand 81 bzw. einer Rückwand 82 derart an das Saugpumpengehäuse 8 anschliessbar, dass eine Seitenwand 71 des Fluidsammelbehälters 7 direkt an eine zwischen den Wandkanten 811 und 821 angeordneten Seitenwand 83 des Saugpumpengehäuses 8 anliegt.

Im Betrieb der Vorrichtung bewirkt die im Saugpumpengehäuse 8 angeordnete Pumpe via einen im Bereich der Seitenwand 83 angeordneten saugpumpengehäuseseitigen Vakuumanschluss 831 und einen in der Seitenwand 71 angeordneten behälterseitigen Vakuumanschluss 711 im Inneren des Fluidsammelbehälters 7 ein Vakuum. Aufgrund dieses Vakuums wird eine Sekretabsaugung durch die Drainageleitung D in das Innere des Fluidsammelbehälters 7 hinein bewirkt.

Zusätzlich zur Drainageleitung D kann eine Hilfsleitung H vorhanden sein, welche vom Saugpumpengehäuse 8 zumindest bis in den Bereich des patientenseitigen Endes der Drainageleitung D führt und zum Beispiel für Druckmessungen oder zum Spülen der Drainageleitung D im Falle einer Verstopfung dienen kann. Die Hilfsleitung H kann, wie es in den Figuren 6a und 6b gezeigt ist, via die Aussenseite des Fluidsammelbehälters 7 über einen in der Seitenwand 83 angeordneten Hilfsanschluss in das Saugpumpengehäuse 8 hinein geführt sein.

Im Bereich der Seitenwand 71 ist am Fluidsammelbehälter 7 eine Peristaltikpumpe 6 ausgebildet. Die Peristaltikpumpe 6 weist einen Pumpenkopf 61 mit mehreren, hier drei Anpressrädern 611 sowie eine umlaufende Anpresswand 62 auf. Zwischen den Anpressrädern 611 und der Anpresswand 62 ist der Schlauch 1 eingeklemmt. Der Schlauch 1 bildet eine Instillationsleitung I.

Bei den Anpressrädern 611 handelt es sich jeweils um Hohlräder, welche via eine Verzahnung von einem zentralen Antriebselement 612 antreibbar sind. Das Antriebselement 612 ist mittels eines Kopplungselements 84 von einem im Saugpumpengehäuse 8 angeordneten Antrieb antreibbar. Die Kopplung zwischen dem Kopplungselement 84 und dem Antriebselement 612 kann insbesondere auf einer magnetischen Kraftübertragung beruhen. Im Betrieb der Vorrichtung wird somit via das Kopplungselement 84 eine Drehbewegung auf das Antriebselement 612 übertragen, welches wiederum die Anpressräder 611 antreibt. Die Anpressräder 611 rollen dadurch auf dem auf der Innenseite der Anpresswand 62 angeordneten Schlauch 1 ab und fördern das darin enthaltene Fluid durch die Instillationsleitung I hindurch zur Wunde des Patienten hin.

Zum Feststellen einer Fluidströmung im Schlauch 1 sind an der Seitenwand 83 des Saugpumpengehäuses Elektroden 31 und 32 eines kapazitiven Sensors 3 angebracht. Um den kapazitiven Sensor 3 optimal vor externen Einflüssen zu schützen, sind die Elektroden 31, 32 vorteilhaft auf der Innenseite der Seitenwand 83 angebracht.

Beim Anschliessen des Fluidsammelbehälters 7 an das Saugpumpengehäuse 8 kommt der Schlauch 1 mit einem Messabschnitt 12 unmittelbar im Bereich des kapazitiven Sensors 3 zu liegen, so dass ein zwischen den Elektroden 31, 32 erzeugtes elektrisches Feld den Messabschnitt 12 mitumfasst. Um eine Fluidströmung im Schlauch 1 detektieren zu können, ist ein Umlenkelement 832 vorgesehen, welche beim Anschluss des Fluidsammelbehälters 7 am Saugpumpengehäuse 8 eine Biegung 14 des Schlauches 1 im Messabschnitt 12 bewirkt. Das Umlenkelement 832 ist hier an der Aussenseite des Saugpumpengehäuses 8 zwischen den beiden Elektroden 31, 32 angebracht, um sicherzustellen, dass der Schlauch 1 erst dann im Messabschnitt 12 umgebogen und damit belastet wird, wenn der Fluidsammelbehälter 7 am Saugpumpengehäuse 8 angeschlossen ist. Als Gegenstück zum Umlenkelement 832 ist an der Aussenseite des Fluidsammelbehälters 7 eine Bogenführung 73 ausgebildet. Beim Anschliessen des Fluidsammelbehälters 7 am Saugpumpengehäuse 8 wird der Schlauch 1 im Messabschnitt 12 zwischen der Bogenführung 73 und dem Umlenkelement 832 eingeklemmt.

Der Schlauch 1 ist in den stromaufwärts und stromabwärts zur Peristaltikpumpe 6 gelegenen Bereichen in einen Schlauchführungskanal 72 eingelegt. Der Messabschnitt 12 ist vorteilhaft, wie es in den Figuren 6a und 6b gezeigt ist, stromabwärts zur Peristaltikpumpe 6 angeordnet. Im Bereich stromabwärts zum Messabschnitt 12 kann der Schlauch 1, also die Instillationsleitung I, entsprechend der in den Figuren 6 und 6b gezeigten Ausführung insbesondere parallel zur Drainageleitung D und zur Hilfsleitung H aus dem Fluidsammelbehälter 7 herausgeführt sein.

Alternative Ausführungsvarianten für den kapazitiven Sensor 3 sind möglich, welche anhand der Figuren 6a bis 6c erläutert werden: Anstatt die Bogenführung 73 sowie das Umlenkelement 832 vorzusehen und Elektroden 31, 32 im Bereich des Umlenkelements 832 anzuordnen, könnten auch Elektroden 31' und 32' vorhanden sein, welche derart an der Seitenwand 83 angeordnet sind, dass sie beim bestimmungsgemässen Anschliessen des Fluidsammelbehälters 7 am Saugpumpengehäuse 8 im Bereich des Pumpenkopfes 61 der Peristaltikpumpe 6 zu liegen kommen. Der Messabschnitt des Schlauches 1 wird dann durch einen an der Anpresswand 62 anliegenden Schlauchabschnitt gebildet. Die Biegung wird durch die Anpressräder 611 bewirkt. Beim Betrieb der Peristaltikpumpe 6 kann dadurch mittels der Elektroden 31', 32' ein periodisches Signal gemessen werden, welches sich bei Vorliegen einer Verstopfung bzw. Ausbleiben einer Fluidströmung verändert. Als weitere Alternative können Elektroden 31" und 32" eines kapazitiven Sensors direkt an der Aussen- oder Innenseite der Anpresswand 62 der Peristaltikpumpe 6 angebracht sein.

In den Figuren 7a und 7b ist eine weitere Ausführungsform einer Vorrichtung mit einer erfindungsgemässen Sensoranordnung gezeigt, wobei es sich hier um eine reine Drainagevorrichtung handelt, die aber allenfalls auch mit einer Instillationsvorrichtung kombiniert werden kann. Die in den Figuren 7a und 7b gezeigte Drainagevorrichtung weist ein Saugpumpengehäuse 8' sowie einen daran anschliessbaren Fluidsammelbehälter 7' auf.

Das Saugpumpengehäuse 8' weist innerhalb einer Seitenwand 83 zwischen zwei vorstehenden Wandkanten 811 und 821 einen zumindest teilweise nach aussen hin vorstehenden Pumpenkopf 61 einer Peristaltikpumpe 6 auf. Die Peristaltikpumpe 6 bildet hier die Saugpumpe. Die Anpressräder 611 der Pumpenkopfes 61 sind durch die vorstehenden Wandkanten 811 und 821 einerseits sowie durch eine flexible Abdeckung 85 vor externen Einflüssen geschützt.

Oberhalb des Pumpenkopfe 61 ist ebenfalls innerhalb der Seitenwand 83, vorteilhaft auf deren Innenseite, ein kapazitiver Sensor 3 mit einem Elektrodenpaar 31, 32 angeordnet. Zwischen den beiden Elektroden 31, 32 ist eine Bogenführung 833 in Form einer in die Seitenwand 83 hinein gewölbten Vertiefung ausgebildet.

Der Fluidsammelbehälter 7' bildet ein Anschlussteil, das derart zwischen den vorstehenden Wandkanten 811, 821 an das Saugpumpengehäuse 8' anschliessbar ist, dass eine Seitenwand 71 des Fluidsammelbehälters 7' direkt an der Seitenwand 83 des Saugpumpengehäuses 8' anliegt.

Ein in das Innere des Fluidsammelbehälters 7' hinein mündender Schlauch 1 bildet eine Drainageleitung D, durch welche hindurch Sekrete von einem Patienten in den Fluidsammelbehälter 7' hinein absaugbar sind. Der Schlauch 1 ist dabei in einen Schlauchführungskanal 72 eingelegt, der in der Seitenwand 71 des Fluidsammelbehälters 7' ausgebildet ist. Beim Anschliessen des Fluidsammelbehälters 7' am Saugpumpengehäuse 8' kommt der derart in den Schlauchführungskanal 72 eingelegte Schlauch 1 derart anliegend an die Abdeckung 85 des Pumpenkopfes 61 zu liegen, dass er zwischen dem Pumpenkopf 61 und dem Schlauchführungskanal 72 eingeklemmt wird. Der Schlauchführungskanal 72 bildet im entsprechenden Bereich eine komplementär zum Pumpenkopf 61 ausgebildete Vertiefung. Wenn der Fluidsammelbehälter 7' somit bestimmungsgemäss an das Saugpumpengehäuse 8' angeschlossen ist und der Pumpenkopf 61 mittels eines im Saugpumpengehäuse 8' untergebrachten Antriebs angetrieben wird, rollen die Anpressräder 611 auf dem Schlauch 1 ab und verursachen dadurch eine Saugwirkung, mit welcher Fluide vom Patienten her durch den Schlauch 1 hindurch in den Fluidsammelbehälter 7' hinein befördert werden.

Beim bestimmungsgemässen Anschliessen des Fluidsammelbehälters 7' an das Saugpumpengehäuse 8' kommt der in den Schlauchführungskanal 72 eingelegte Schlauch 1 ausserdem mit einem Messabschnitt 12 unmittelbar in den Bereich zwischen den Elektroden 31, 32 des kapazitiven Sensors 3 zu liegen. Mittels eines im Schlauchführungskanal 72 an der entsprechenden Stelle angeordneten Umlenkelements 75 wird der Schlauch 1 dabei in die Bogenführung 833 hineingedrückt. Der Schlauch 1 bildet dadurch im Messabschnitt 12 eine Biegung 14, welche sich in den Bereich zwischen den beiden Elektroden 31, 32 hinein erstreckt. Mittels des kapazitiven Sensors 3 ist es dadurch möglich, das Vorhandensein eines Fluidstroms oder einer Verstopfung im Schlauch 1 zu detektieren.

Wenn ausgehend von der Antriebsgeschwindigkeit des Pumpenkopfes 61 die ungefähre Durchflussrate im Schlauch 61 bekannt ist bzw. abgeschätzt werden kann, ist es möglich, mit Hilfe der in den Figuren 7a und 7b gezeigten Sensoranordnung auf die abgesaugte und im Inneren des Fluidsammelbehälters 7' gesammelte Menge an Fluid bzw. Sekret zu schliessen. Es braucht hierfür lediglich die Zeitdauer ermittelt zu werden, während welcher ein Fluidstrom im Schlauch 1 vorliegt, nachdem ein leerer Fluidsammelbehälter 7' an das Saugpumpengehäuse 8' angeschlossen wurde. Mit Hilfe des kapazitiven Sensors 3 ist eine derartige Ermittlung problemlos möglich. Die Bestimmung der Sekretmenge im Fluidsammelbehälter 7' auf diese Weise ist insbesondere dann von Vorteil, wenn im Fluidsammelbehälter 7' ein Gelier- oder Verdickungsmittel vorhanden ist, um die abgesaugten Sekrete zu binden. Mit herkömmlichen Methoden, bei denen beispielweise mittels einer kapazitiven Messung der Füllstand im Fluidsammelbehälter 7' bestimmt wird, besteht das Problem, dass das Sekret im Inneren des Behälters 7' aufgrund des Gelier- oder Verdickungsmittels Klumpen bildet und dadurch keine ebene Oberfläche vorliegt, anhand welcher ein Füllstand abgelesen werden könnte. Eine auf dem Füllstand beruhende Messung der abgesaugten Sekretmenge kann dadurch sehr ungenau werden.

Alternativ oder zusätzlich kann insbesondere in Fällen, wo kein Gelier- oder Verdickungsmittel im Fluidsammelbehälter enthalten ist, eine Skala 74 auf der Aussenseite des Fluidsammelbehälters 7' aufgedruckt sein, mittels welcher der Füllstand abgelesen werden kann. Der Fluidsammelbehälter 7' sollte dann zumindest im Bereich der Skala 74 transparent ausgebildet sein.

In den Figuren 8a und 8b ist eine Ausführungsform gezeigt, bei welcher eine Biegung 14 in Form eines Knicks innerhalb des Messabschnitts 12 des Schlauches 1 dadurch erzeugt wird, dass der Schlauch 1 an einem Anschlussstutzen 12 angeschlossen ist, der sich in Richtung zu einem hier zapfenförmigen Umlenkelement 43 hin erstreckt. Dabei erstreckt sich der Anschlussstutzen 48 insbesondere geneigt zur Wandung, innerhalb welcher er angeordnet ist, sowie geneigt zur Haupterstreckungsrichtung des Schlauches 1 in den Bereichen ausserhalb des Messabschnitts 12.

**BEZUGSZEICHENLISTE**

| | | | |
|---|---|---|---|
| 1 | Schlauch | 6 | Peristaltikpumpe |
| 11 | Eingangsabschnitt | 61 | Pumpenkopf |
| 12 | Messabschnitt | 611 | Anpressrad |
| 13 | Ausgangsabschnitt | 612 | Antriebselement |
| 14 | Biegung | 62 | Anpresswand |
| | | | |
| 21 | Fluid | 7, 7' | Fluidsammelbehälter |
| 22 | Luft | 71 | Seitenwand |
| 23 | Pfropfen | 711 | Vakuumanschluss |
| | | 72 | Schlauchführungskanal |
| 3 | Kapazitiver Sensor | 73 | Bogenführung |
| 31, 31', 31" | Erste Elektrode | 74 | Skala |
| 32, 32', 32" | Zweite Elektrode | 75 | Umlenkelement |
| 33 | Feldlinien | | |
| | | 8, 8' | Saugpumpengehäuse |
| 4, 4' | Schlauchführungsvorrichtung | 81 | Vorderwand |
| 41 | Schlauchführung | 811 | Vorstehende Wandkante |
| 42 | Führungselement | 82 | Rückwand |
| 43 | Umlenkelement | 821 | Vorstehende Wandkante |
| 44 | Bogenführung | 83 | Seitenwand |
| 45 | Einspannhebel | 831 | Vakuumanschluss |
| 451 | Zapfenaufnahme | 832 | Umlenkelement |
| 452 | Einrasthaken | 833 | Bogenführung |
| 46 | Gelenkzapfen | 84 | Kopplungselement |
| 47 | Einrastzapfen | 85 | Abdeckung |
| 48 | Anschlussstutzen | | |
| | | D | Drainageleitung |
| 51 | Erstes Rohrwinkelstück | H | Hilfsleitung |
| 52 | Zweites Rohrwinkelstück | I | Instillationsleitung |

## Patentansprüche

1. Sensoranordnung zum Detektieren einer Fluidströmung bei einer Vorrichtung zum Zuführen und/oder Absaugen einer fluiden Substanz (21) zu oder von einem menschlichen oder tierischen Körper, aufweisend
einen Schlauch (1) mit einem Messabschnitt (12); sowie
einen kapazitiven Sensor (3) zum Detektieren einer Fluidströmung im Messabschnitt (12);
wobei der Schlauch (1) innerhalb des Messabschnitts (12) zumindest eine Biegung (14) aufweist.

2. Sensoranordnung nach Anspruch 1, wobei der kapazitive Sensor (3) zumindest eine ausserhalb des Schlauches (1) angeordnete Elektrode (31, 32) aufweist.

3. Sensoranordnung nach Anspruch 1 oder 2, wobei der gesamte kapazitive Sensor (3) ausserhalb des Schlauches (1) angeordnet ist.

4. Sensoranordnung nach einem der Ansprüche 1 bis 3, wobei der kapazitive Sensor (3) mehr als eine, insbesondere mehr als zwei, Elektroden (31, 32) aufweist.

5. Sensoranordnung nach einem der vorhergehenden Ansprüche, wobei der Schlauch (1) im Messabschnitt (12) derart elastisch ausgebildet ist, dass aufgrund der Biegung (14) die Menge an Fluid im Messabschnitt (12) bei Unterdruck deutlich geringer als bei Normaldruck ist.

6. Sensoranordnung nach einem der vorhergehenden Ansprüche, wobei der Schlauch (1) innerhalb des Messabschnitts (12) mehrere Biegungen (14) aufweist.

7. Sensoranordnung nach einem der vorhergehenden Ansprüche, wobei es sich bei der Biegung (14) um einen Knick handelt.

8. Sensoranordnung nach einem der vorhergehenden Ansprüche, zusätzlich aufweisend ein Umlenkelement (43; 75; 832) zur Herstellung der Biegung.

9. Sensoranordnung nach Anspruch 8, wobei das Umlenkelement (43; 75; 832) relativ zu derjenigen Stelle, wo der Schlauch (1) die zumindest eine Biegung aufweist, bewegbar ist, um den Schlauch (1) bei der Lagerung zu entlasten.

10. Sensoranordnung nach einem der vorhergehenden Ansprüche, wobei die Biegung (14) durch die Anpressrolle (611) einer Peristaltikpumpe (6) bewirkt wird.

11. Anschlussteil (4; 4'; 7; 7') zum Anschliessen an eine Vorrichtung zum Zuführen und/oder Absaugen einer fluiden Substanz (21) zu oder von einem menschlichen oder tierischen Körper, aufweisend einen Schlauch (1) sowie eine Schlauchführung (41; 72) zum Führen des Schlauches (1) derart, dass der Schlauch (1) zumindest eine Biegung (14) aufweist und dadurch einen Messabschnitt (12) zum Detektieren einer Fluidströmung mittels eines kapazitiven Sensors (3) bildet.

12. Anschlussteil nach Anspruch 11, wobei es sich beim Anschlussteil um einen Fluidsammelbehälter (7; 7') zur Aufnahme der abgesaugten fluiden Substanz (21) handelt.

13. Anschlussteil nach Anspruch 12, wobei der Schlauch (1) in das Innere des Fluidsammelbehälters (7') mündet und dadurch als Teil einer Drainageleitung (D) vorgesehen ist, durch welche hindurch die abgesaugte fluide Substanz (21) in den Fluidsammelbehälter (7') hinein gefördert wird.

14. Anschlussteil nach Anspruch 11 oder 12, zusätzlich aufweisend eine Peristaltikpumpeneinheit (6), wobei der Schlauch (1) ein Teil dieser Peristaltikpumpeneinheit (6) darstellt, so dass die fluide Substanz (21) mittels der Peristaltikpumpeneinheit (6) durch den Schlauch (1) hindurch zum menschlichen oder tierischen Körper hin gefördert oder von diesem abgesaugt werden kann.

15. Vorrichtung zum Zuführen und/oder Absaugen einer fluiden Substanz (21) zu oder von einem menschlichen oder tierischen Körper, aufweisend
ein Pumpengehäuse (8; 8') zur Aufnahme einer Pumpe; sowie
ein Anschlussteil (7; 7') nach einem der Ansprüche 11 bis 14, zum Anschliessen an das Pumpengehäuse.

16. Vorrichtung nach Anspruch 15, wobei das Pumpengehäuse (8) ein Umlenkelement (832) aufweist, welches beim Anschliessen des Anschlussteils (7) an das Pumpengehäuse (8) die zumindest eine Biegung (14) des Schlauches (1) bewirkt.
